# EUROPEAN PATENT SPECIFICATION

(11) **EP 2 429 467 B1**
(45) Date of publication and mention of the grant of the patent: **16.07.2014**
(21) Application number: 10719989.5
(22) Date of filing: 12.05.2010
(51) Int. Cl.: A61F 5/44

(54) **URINE COLLECTION BAG**
URINAUFFANGBEUTEL
SAC COLLECTOR D'URINE

(30) Priority: 12.05.2009 DK 200900607; 15.07.2009 DK 200970064
(43) Date of publication of application: 21.03.2012
(73) Proprietor: Coloplast A/S, 3050 Humlebæk (DK)
(72) Inventor: TANGHOEJ, Allan, DK-2980 Kokkedal (DK)
(86) International application number: PCT/DK2010/050102
(87) International publication number: WO 2010/130261

(56) References cited:
- EP-A1- 0 748 620
- DE-A1- 2 249 132
- DE-A1- 2 936 622
- FR-A1- 2 326 208
- US-A1- 2009 077 734

## Description

The invention relates to a urine collection bag for use in connection with a catheter, the urine collection bag of which is provided with an anti-reflux valve at the inlet. The invention also relates to a kit of parts comprising a urine collection bag and a catheter.

### Background

For a large group of persons, intermittent catheterisation is a daily life procedure that takes place several times a day. Typically, catheters for intermittent catheterisation are used by people suffering from urinary incontinence or by disabled individuals like para- or tetraplegics who may have no control permitting voluntary urination, and for whom catheterisation may be the way of urinating. Using an intermittent catheter, the bladder may be drained through a natural or artificial urinary canal.

The availability of catheter assemblies, which are compact and discreet to transport and dispose of, in addition to being easy to use, even for individuals with reduced dexterity, significantly improves quality of life for a large group of individuals. SpeediCath® Compact Female is a compact catheter, as described above, and is produced by Coloplast A/S.

In some situations, a user may wish to use a catheter with a urine bag connected to it, for example when a patient in a wheel chair is unable to move from the wheel chair to the toilet. Urine bags are typically made from sheets of a foil material joined along the edges. The thickness of the urine bag is therefore very small, but due to the plane dimensions of the urine bag, it is rather indiscreet.

### Description of related art

EP0748620 A1, which is regarded as the closest prior art, describes a urine collection bag with an insert at an inlet, the insert of which comprises two lamellas which are relatively rigid, yet flexible. The insert further comprises two tubular elements of the same foil material as the bag extending inside the bag. One of these tubular elements extends beyond the other. At the end of each element, the element is welded to itself at discrete points.

DE 22 49 132 discloses a disposable uniral comprising a water proof container with an inlet and an outlet which is smaller than the inlet.

DE 29 36 622 discloses a single use urine bag made from plastic film, with a bag inlet valve which consists of an inlet funnel made of two sheets

US 2009/077734 discloses a personal disposable sanitary toilet system that includes a primary toilet/containment bag and a secondary containment bag to minimize odor, vapor and liquid leakage. Both bags may be made out of sheet-like liquid impermeable material such as a plastic film or multi-layer plastic film.

### Summary of the Invention

The invention relates to a urine collection bag for use in connection with a catheter. The urine collection is made of foil layers and comprises a one-way valve (an anti-reflux valve) at the inlet. The valve is relatively long compared to the width which is contemplated to help keeping the valve closed unless liquid enters from the opening into the inlet and thereby into the valve. An aspect of the invention relates to where the inlet and valve are adapted in width to an outlet from a urinary catheter. Thereby the valve fits snugly at the outlet such that spillage from the catheter should be minimized. The invention also relates to a catheter kit comprising a catheter and a urine collection bag.

### Detailed Description of the Invention

In a first aspect, the invention concerns a urine collection bag comprising
- an end with an opening at the top and a closed bottom at an opposite end defining a longitudinal direction of the bag between the opening and the bottom and a transverse direction of the bag transverse to the longitudinal direction,
- an inlet at the opening extending from the opening and a length into the bag,
- the inlet being an anti-reflux valve in form of a foil-valve with four layers of foil welded together in the longitudinal direction defining a width of the inlet between the welds in the transverse direction and the length of the inlet as a length of the foils in the longitudinal direction,
- wherein a most narrow width is adapted for use with a urinary catheter having a connector,
- wherein the material of the urine collection bag is slightly elastic so that the most narrow width creates a narrow fit around the connector of the catheter.

Urinary catheters may be provided with a connector at the outlet of the catheter. In this situation, the width is adapted so that the connector is able to enter into the valve, hence the inlet of the bag. In an embodiment, the length is at least 10 cm. Typically, the connector has a standard size, for example between 10 and 15 mm.

Another way of describing this is that the valve or the inlet of the urine collection bag is configured to receive a catheter with an outlet of a standard size. In particular the urine collection bag is configured to receive a catheter of the type SpeediCath Compact Female as produced by Coloplast A/S. When a catheter of this type is inserted into the inlet of the bag, the inlet will provide a narrow fit around the outlet. This way the outlet is sealed with respect to the collection bag. The outlet of SpeediCath Compact Female is slightly conical and has a largest diameter of approximately 10 mm to 12 mm. The outlet of a standard catheter (the connector) is also conical and has a largest diameter of substantially the same size but may be slightly larger - up to 15 mm.

Both types of catheters fit within the inlet of the bag. A compact catheter such as SpeediCath Compact Female catheter will be able to be entered completely into the bag after it has been used. Thereby the parts to be disposed of are kept together, and the user does not have to pack the catheter and bag separately, which is especially convenient when the products cannot be readily disposed of.

The bag is made of a foil material such as low-density polyethylene (LDPE), which may be blended with ethyl vinyle acetate (EVA). LDPE provides a foil with a slight elasticity so that it springs back to its original configuration when extended slightly. When LDPE is blended with EVA, the foil material provides more surface friction and hence stronger closure of the valve. A slight elasticity of the material may be an advantage in connection with entering an outlet from a catheter into the inlet and valve. When the outlet is entered, it may slightly dilate the diameter of the valve, and due to the elasticity of the material, the valve will contract again to fit tightly around the outlet of the catheter. An example of material suited for the urine collection bag is a polyethylene film of VLLDPE (very low density polyethylene), such as type 98826 from Ulfoss Plastic A/S. The elasticity of the material may be expressed by the modulus of elasticity of the material. The modulus of the abovementioned film may be between 88.5 and 171 MPa according to ASTM D882, depending on the polymer direction compared to the pulling direction. A film - that is a material for the urine collection bag - may have a modulus of elasticity between 80 MPa and 180 MPa when determined according to ASTM D882. Such a film will be suitable for use.

The urine collection bag described above according to a first or second aspect of the invention may in one embodiment have a relationship between a most narrow width of the inlet and the length of the inlet such that the length is less than 3.5 times the width. In a related embodiment, the length is between 4 cm and 8 cm. Such a length will enable the outlet of the catheter placed in the valve to keep the valve open all the way to the end of it. Thereby urine exiting through the catheter and the outlet will unhindered enter into the collection bag.

The urine collection bag may in another embodiment have a relationship between a most narrow width of the inlet and the length of the inlet such that the length is more than 4 times the width. In a related embodiment, the length is more than 10 cm. If the valve is so long, then the catheter outlet placed in the valve will not be able to keep the valve open all the way to the end of it. Thereby, urine flowing from the bag towards the catheter will be prevented from flowing back into the catheter and further into the bladder. The bag is not a sterile product. Therefore, urine which has been in contact with the bag may be contaminated. Thus it should be avoided that urine returns into the bladder. By having an extra long valve, the back-flow is prevented - even if the user were to hold the bag above the catheter outlet.

The urine collection bag comprises a collecting lumen at an end of the bag. The opening into the bag is placed opposite the collecting lumen in the longitudinal direction of the bag. When the bag is held in the normal use-position the opening is uppermost and the collecting lumen is lowermost. The top of the bag is defined as the end including the opening and the bottom as the opposite end. In the normal use-position, the normal flow direction will be downwards, such that the collecting lumen is placed below the opening. When referring to below or above, the referral is in line with the normal flow direction. The width direction of all parts of the bag is defined as the direction transverse to the longitudinal direction - that is the direction transverse to the normal flow direction. The longitudinal direction of all parts of the bag is defined as the direction from top to bottom - that is the direction along the normal flow direction.

The foil-valve is made of four layers of foil welded together in the longitudinal direction leaving a gap between them. This gap functions as the inlet. At the opening, the four layers are welded together two and two in the transverse direction leaving two flaps extending into the bag at the inside. With this configuration liquid (in this case urine) can flow into the opening and through the gap (the inlet) and further down below the foil-valve. When the foil-valve is turned upside down, the two free flaps will close the gap between them so as to close the inlet. Thereby liquid (urine) cannot exit the bag.

The material for the bag is flexible and bendable so that it may be packed (for example by folding) in a configuration taking up as little space as possible. The bag can easily be folded into a configuration that is small enough to fit in a small bag or a hand.

Because the bag is made of only a few pieces of foil, only a small amount of material is used to make a bag. No further attachments need to be fitted into the bag. Furthermore, a foil bag consisting essentially only of welded foil layers, is cheap and easy to produce.

The urine collection bags may be provided with two flaps at the inlet for tying a knot around the inlet. Such flaps tied in a knot provide an increased security against spilling of urine. A knot tied with these flaps is an advantage in cases where a user has used the bag in a place where it cannot be emptied and disposed of - for example when the bag has been used in another room than a toilet or bathroom. When the flaps are tied at the inlet, the bag is completely closed and no urine can flow out. The user now has the possibility of transporting the bag to a place where it can be emptied and disposed of.

In an embodiment, the flaps are attached to the urine collection bag at tearable joints prior to use. Thereby the flaps on the bag are always available for use. In a related embodiment, the tearable joints are provided as perforation lines. These perforations may extend substantially along the inlet to a point below the opening of the bag. Thereby, when the perforations are torn, the flaps are still attached to the bag and readily provide for the possibility of tying a knot at the inlet.

The collection bag may be provided with holes for hanging or holding the bag. Such holes are typically provided at the top of the bag. The holes for hanging or holding have a curvature, and the tearable joints at the flaps may terminate in this curvature. Due to the curvature, the flaps will not have a direction for tearing further at the inlet.

Another embodiment relates to the foil valve which is provided with constrictions on the sides of the valve. By the sides of the valve is meant the longitudinal direction of the welds facing the inlet of the bag. The constrictions function as a stop for an outlet from a catheter so that when the catheter is inserted in the inlet - and thereby in the valve - the user will feel a certain resistance at the constrictions. In this way, the user will know that the outlet is inserted adequately. Two or more sets of constrictions may be provided. The first set of constrictions may be placed approximately 2 cm below the inlet of the bag.

If the length of the valve is less than 3.5 times the width, this position of the constrictions corresponds to 0.8-0.9 of the length of the valve, below the opening. In other words the constrictions are placed closer to the lower end of the valve than to the opening into the valve. When the constrictions are placed at this point, then the outlet of the catheter is able to keep the valve open all the way to the length. This may be an advantage because the flow of urine will always be able to flow into the bag as it is open beneath the catheter in the normal flow direction. The normal flow direction is defined as a flow direction from the opening of the bag, through the valve and into the bag.

If the length is more than 4 times the width, the position of the constrictions corresponds to less than half the length of the valve such as 0.3-0.4 times the length. Using the definition above for the normal flow direction, the constrictions are placed less than halfway through the valve in the normal flow direction. When the constrictions are placed at this point, then the outlet of the catheter is prevented from keeping the valve open all the way to the end of the valve. When the catheter is to be inserted, the user fits the catheter in the bag at the constriction. During insertion of the catheter into the urethra, the bag may be kept in a higher position than the catheter. By having the constrictions at that position the valve below the catheter in the normal flow direction will open for urine entering into the bag in the normal flow direction, but will be closed for urine coming from the bag - that is, against the normal flow direction - even with catheter placed in the valve at the constrictions.

In an embodiment, the urine collection bag is provided with a tear tab for opening into an outlet from the bag. The tear tab may be sized so that a user can grab and pull it with two fingers so it opens into an outlet. The outlet may be placed in the top part of the bag. When holding the bag at the top and tearing it open by the tear tab into the outlet, urine will not flow out of the bag.

The bag may be provided with at least one lobe for holding or handling the bag during use and emptying. In an embodiment, the holding lobe(s) may be provided with a hole for hanging the bag. In some situations, for example when tying knots, emptying, tearing the tab, and so on, it may be an advantage to have such a lobe on the bag. It may be difficult to handle a bag filled with liquid without having a lobe or other part to hold. Users who have poor hand dexterity may find it advantageous that the holding point is provided with a hole for hanging the bag. They can then hang the bag from a hook or similar means when they need to handle it. One of the holding lobes may be placed opposite the outlet of the bag. This holding lobe may then be used for holding or hanging the bag while it is emptied.

Another aspect of the invention relates to a kit of parts comprising a catheter and a urine collection bag as described above.

In an embodiment, the catheter is of the type SpeediCath Compact Female as distributed by Coloplast A/S.

Such a kit of parts can be packed and stored together taking up very little space which may be an advantage for the user when travelling or being away from home in general. In a kit of parts, the outlet of the catheter and the inlet of the bag are designed to fit together so that the catheter is closely connected to the bag when it is used. This configuration helps prevent spillage because the parts are able to stay connected even if the user should lose grip of one of the parts. All the liquid exiting the catheter is "forced" to flow into the bag.

### Brief description of the drawing

- Figure 1-3 illustrates a first embodiment of a urine collection bag according to the invention.
- Figure 4-6 illustrates a second embodiment of a urine collection bag according to the invention.
- Figure 7 illustrates how a catheter is inserted in the anti-reflux valve.
- Figures 8 and 9 illustrate in cross-section the cooperation between the catheter and the anti-reflux valve in the first and second embodiment respectively.
- Figure 10 illustrates other details of the bag.
- Figure 11 illustrates a bag with pictograms on the side of the bag, describing how it is used.

### Detailed description of the drawing

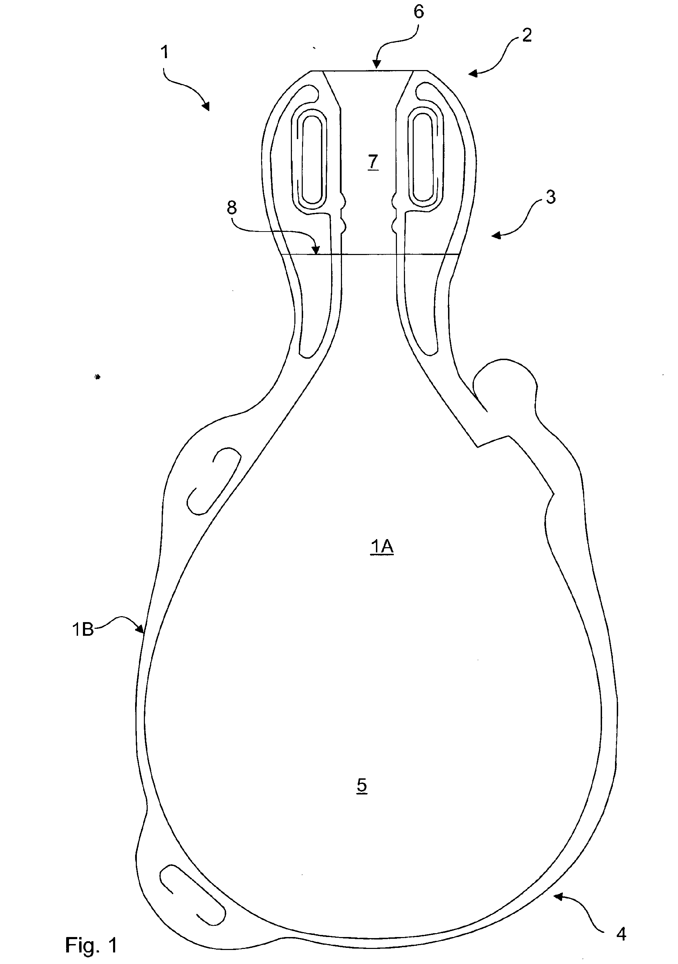

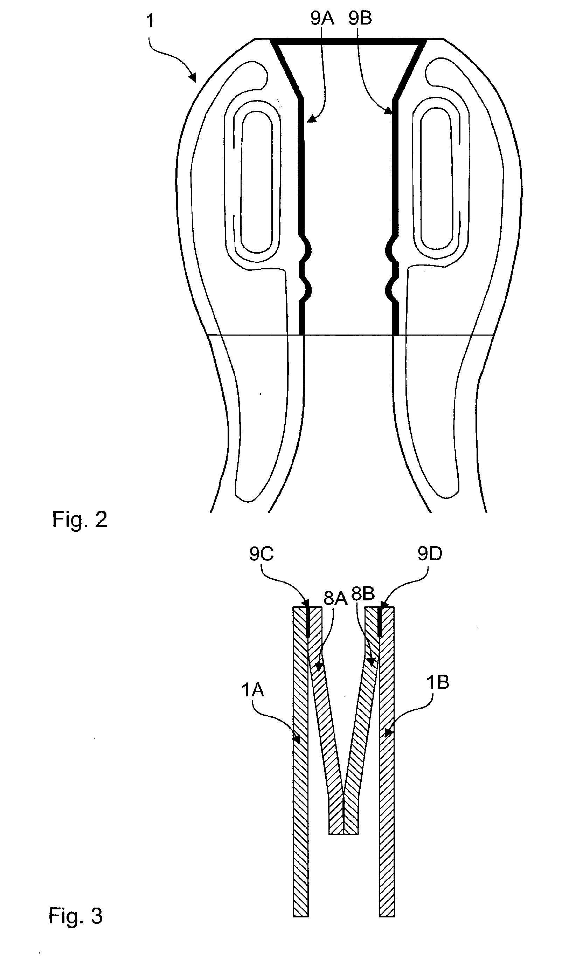

Figures 1-3 illustrate a first embodiment of a urine collection bag 1 according to the invention. This urine collection bag **1** is made of two foil layers **1 A, 1B** and is generally pear-shaped with a top end **2** with a neck section **3** and a bottom end **4** with a wider collecting lumen **5.** A pear-shape provides a bag with a low centre of gravity whereby the bag will be prone to be in a position with the top end facing upwards. The two foil layers 1 **A** and **1B** are welded together along their outer contour so that it constitutes a closed bag. An inlet 6 to the bag **1** is placed in the neck section **3.** This inlet **6** is made as an antireflux valve **7** made of four layers of foil, two of which are constituted by the foil layers **1 A** and **1B** of the bag. The anti-reflux valve **7** extends into the bag as indicated by the transverse line **8** showing the length of the extra foil layers, **8A** and **8B,** see figure 1 and 3. The anti-reflux valve is made by welding the four layers of foil together along the sides of the inlet, as indicated at **9A** and **9B** in figure 2, and at the top, as indicated at 9C and 9D in figure 3. In figure 3, the cross-section of the foil layers is exaggerated to elucidate how the layers work together.

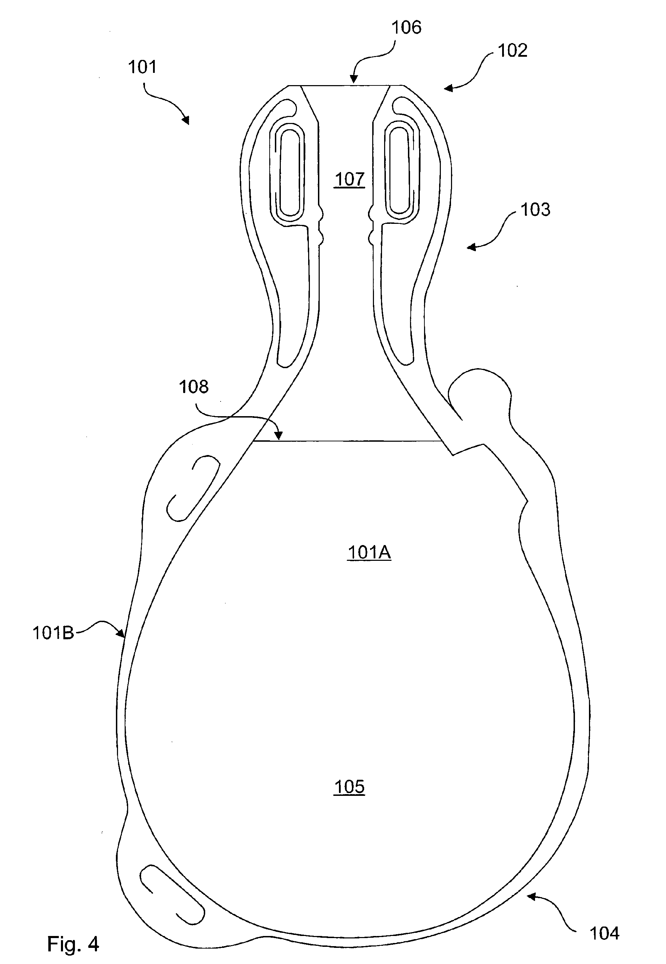

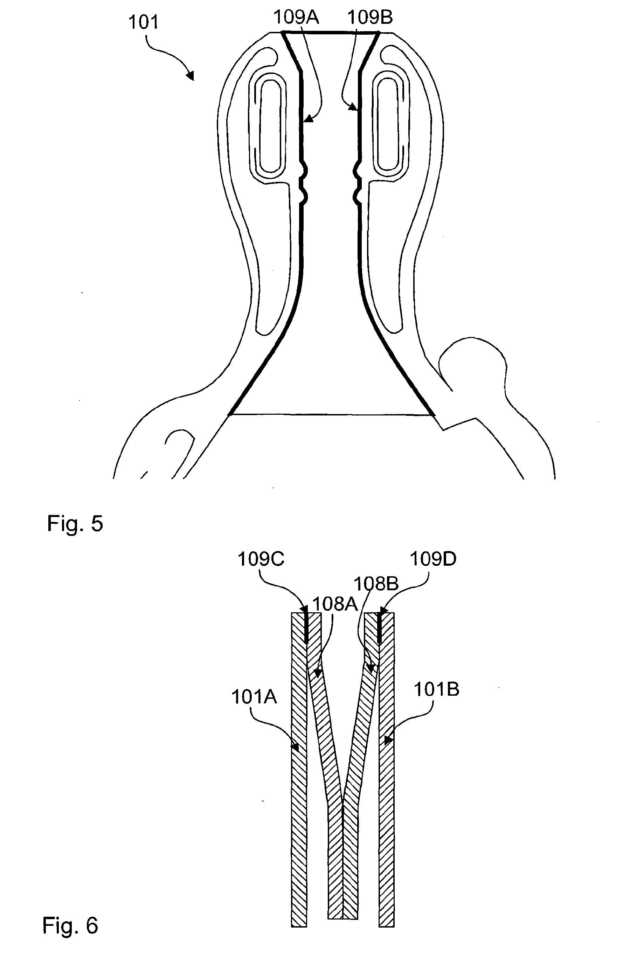

Figures 4-6 illustrate a second embodiment of a urine collection bag **101** according to the invention. In this embodiment, the anti-reflux valve **107** is longer. Otherwise, the embodiment corresponds to the first embodiment. The objects in figures 4-6 are numbered like in figures 1-3 except for the prefix **100.** That the anti-reflux valve **107** is longer appears from figure 4 by the positioning of the transverse line **108** and in figure 6, by the longer closing of the two foil layers **108A** and **108B.**

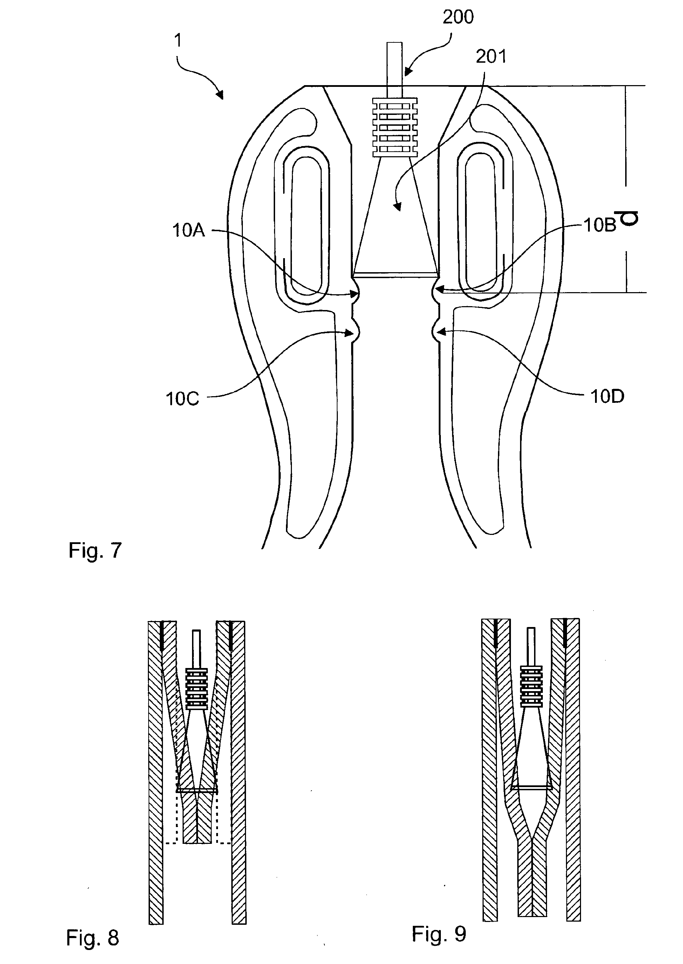

Figure 7 illustrates a catheter **200** with a regular connector **201** placed in the inlet **6.** The inlet **6** and the valve **7** comprise constrictions **10A** to **10D** at the sides of the inlet **6** and valve **7.** These constrictions have the function that when the outlet end of a catheter, for example a connector end, meets these constrictions, the user will not be able to insert the catheter further or will at least feel a slight resistance. Thus, when the outlet end is placed at the constrictions, the catheter is placed correctly in the bag. The constrictions are placed distance *d* from the end of the valve as indicated in the figure. The positioning of the constrictions with respect to the top of the bag is the same irrespective of whether the urine collecting bag is a bag of the type like the first embodiment illustrated in figures 1-3 or the type like the second embodiment illustrated in figures 4-6. That is the distance d is the same for the first and second embodiment.

Figure 8 illustrates in cross section the behaviour of urine collection bag **1** of the first embodiment type, when the catheter **200** is placed in the inlet **6.** In this embodiment, when the outlet is at the first constriction from the inlet **10A, 10B,** the valve **7** will be open below the connector **201.** Therefore, the urine will be able to flow freely into the bag. In figure 8, the cross-section of the foils is exaggerated when compared to other dimensions in the figure. The dashed lines of the foils indicate the position of the foils with the catheter **200** inserted in the inlet **6.**

Figure 9 illustrates also in cross section, the behaviour of a urine collection bag **101** of the second embodiment, when the catheter **200** is placed in the inlet **106.** In this embodiment, the valve **107** will be closed below the connector **201** and will only be open when urine is flowing. This will prevent urine from the bag from entering into the catheter and further on into the bladder. Again, the cross-section of the foils is exaggerated when compared to other dimensions in the figure.

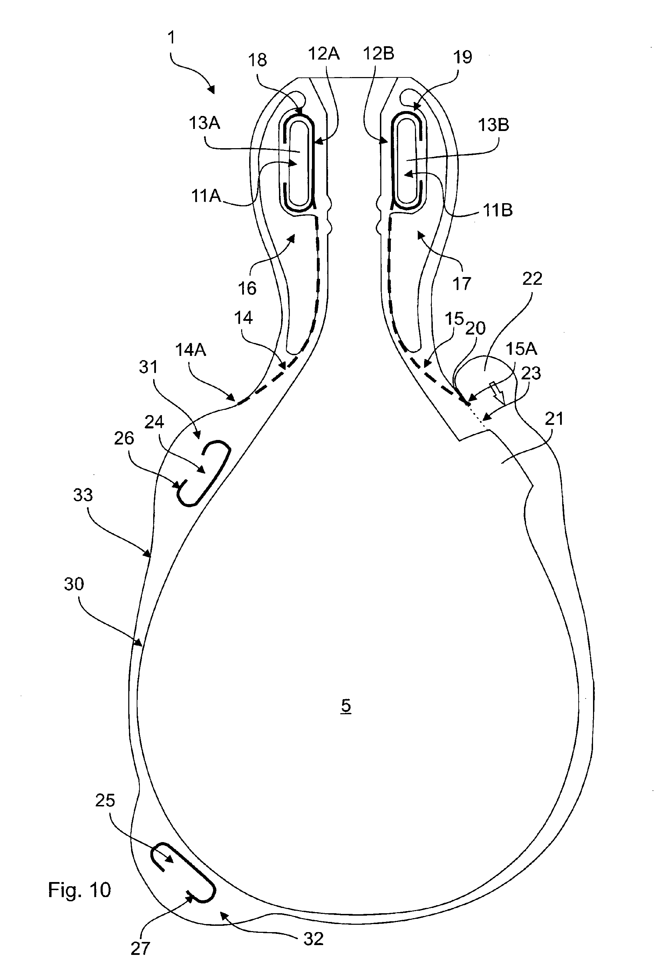

Figure 10 illustrates other details on the urine collection bag. These details may be provided on a bag according to the first embodiment or the second embodiment. Thus the length and configuration of the anti-reflux valve is not shown in the figure. Figure 10 illustrates the provision of two holes **11 A** and **11B** at the top end **2** of the bag. These two holes **11 A** and **11B** may be used for hanging or holding the bag **1.** The two holes **11 A, 11B** may be made by welding around them and then providing a cut along the lines **12A, 12B.** Thereby two hole-flaps **13A, 13B** are provided. These two hole-flaps may be pivoted in directions out of the plane of the drawing whereby the two holes 11 **A, 11B** are created.

The bag of figure 10 has two perforation lines **14, 15.** These two perforation lines **14, 15** may be used for providing flaps **16, 17** for tying a knot around the inlet 6. The perforation lines **14, 15** extend from one side of the bag **14A, 15A** and terminate at the cut-lines **12A, 12B** so that when the perforation lines are torn, the flaps **16, 17** are connected to the bag in a rounding **18, 19.** Thereby there will be no direction for a rupture line between the flaps **16, 17** and the bag **1.**

Furthermore, the cutting process may also provide a predefined cut **20** for tearing open into an outlet **21** by pulling at a tab **22.** When the tab **22** is pulled in the arrow's direction, the predefined cut **20** will function as a direction for a tear line and extend as indicated by the dotted line **23.** Thereby the outlet **21** will eventually be opened, and the liquid contained in the bag **1** can be poured out.

Finally, two additional side holes **24, 25** may be provided in the same cutting process. The side holes **24, 25** may be made again by providing a cut line **26, 27** around a hole-flap. The first side hole **24** may be used for holding the bag during tearing of the tab **22.** The second side hole **25** may be used for holding the bag while pouring out the liquid contained in the bag. The two side holes **24, 25** are placed outside the inner contour **30** defining the collecting volume 5 of the bag. They are placed in lobes **31, 32** of welded foil layers extending from the inner contour **30** such that the outer contour 33 are not congruent with the inner contour **30.** Likewise, the tab **22** is a lobe of welded foil layers extending from the inner contour.

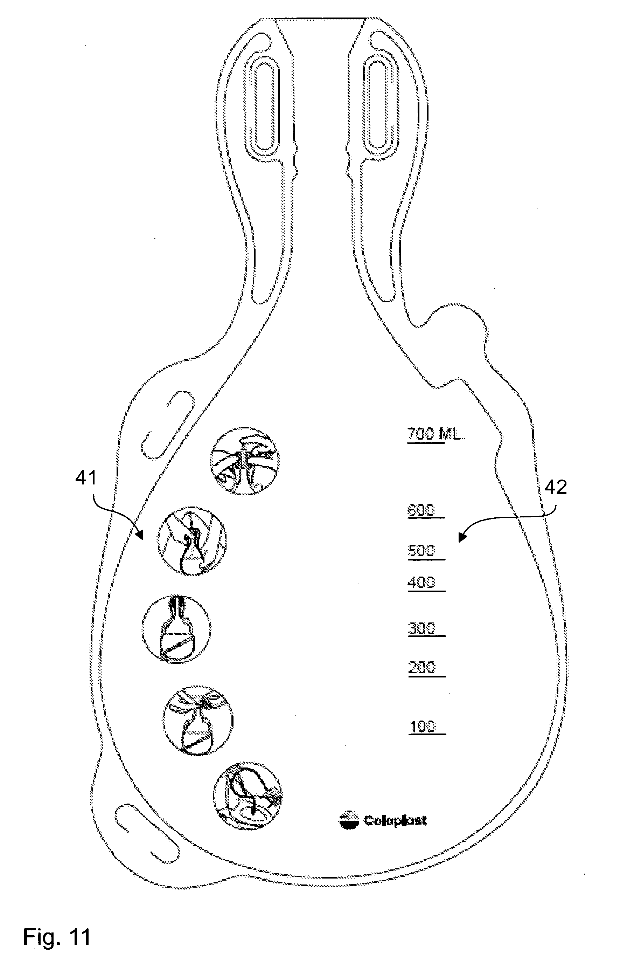

Figure 11 illustrates an embodiment of the urine collection bag where the bag is provided with pictograms **40** on one side of the bag, showing instructions for use. Furthermore, the bag may be provided with a scale **41** for indicating the volume contained in the bag. Again, these two features may be provided on a bag according to a first embodiment as well as on a bag according to a second embodiment of the invention.

## Claims

1. A urine collection bag (1,101) comprising:
- an opening defined at a top end (2,102) and a closed bottom defined at an opposite bottom end (4,104), wherein a longitudinal direction of the bag (1,101) is defined between the opening and the closed bottom and wherein a transverse direction of the bag is defined in a direction transverse to the longitudinal direction; and
- an inlet (6,106) which is defined at the opening and which extends from the opening and a length into the bag;
wherein the inlet (6,106) is an anti-reflux valve (7,107) in form of a foil-valve with four layers of foil welded together in the longitudinal direction, wherein a width of the inlet is defined in the transverse direction between the welds and wherein a length of an inlet is defined as a length of the foils in the longitudinal direction;
**characterised in that**
a most narrow width of the inlet (6,106) is adapted for use with a urinary catheter (200) comprising a connector (201), and **in that** the material of the urine collection bag (1,101) is slightly elastic so that the most narrow width creates a narrow fit around the connector (201) of the catheter (200).

2. The urine collection bag according to claim 1, wherein the material of the urine collection bag (1,101) has a modulus of elasticity between 80 MPa and 180 MPa when determined according to ASTM D882.

3. The urine collection bag according to any of claims 1 to 2, wherein the relationship between a most narrow width of the inlet (6,106) and the length of the inlet (6,106) is such that the length is less than 3.5 times the width.

4. The urine collection bag according to claim 3, wherein the length is between 4 cm and 8 cm.

5. The urine collection bag according to any of claims 1 to 2, wherein the relationship between a most narrow width of the inlet (6,106) and the length of the inlet (6,106) is such that the length is more than 4 times the width.

6. The urine collection bag according to claim 5, wherein the length is more than 10 cm.

7. The urine collection bag according to any preceding claims, wherein the bag (1,101) is provided with two flaps at the inlet (6,106) for tying a knot around the inlet (6,106).

8. The urine collection bag according to claim 7, wherein the flaps are attached to the collection bag (1,101) at tearable joints prior to use.

9. The urine collection bag according to claim 8, wherein the tearable joints are provided as perforation lines.

10. The urine collection bag according to any of claims 8 or 9, wherein the tearable joints terminate in holes provided on the bag adapted for hanging or holding the bag (1,101).

11. The urine collection bag according to any preceding claims, wherein the foil valve is provided with constrictions at a side of the valve.

12. The urine collection bag according to claim 11, wherein constrictions are placed at a position corresponding to 0.8-0.9 of the length of the valve below the opening of the bag.

13. The urine collection bag according to claim 11, wherein constrictions are placed less than half of the length of the valve below the opening of the bag

14. The urine collection bag according to any preceding claims, wherein the bag is provided with a tear tab for opening into an outlet (6,106) from the bag.

15. The urine collection bag according to any preceding claims, wherein the bag (1,101) is provided with at least one holding lobe for holding or handling the bag (1,101) during use and emptying.

16. The urine collection bag according to claim 15, wherein the holding lobe(s) is provided with a hole for hanging the bag (1, 101).

17. The urine collection bag according to claim 15 or 16, wherein one of the holding lobes is placed opposite the outlet from the bag (1,101).

18. Kit of parts including a urine collection bag as claimed in any of claims 1-17, and a catheter (200) having an outlet part.

## Patentansprüche

1. Urinsammelbeutel (1, 101), umfassend:
- eine an einem oberen Ende (2, 102) definierte Öffnung und einen an einem gegenüberliegenden unteren Ende (4, 104) definierten geschlossenen Boden, wobei eine Längsrichtung des Beutels (1, 101) zwischen der Öffnung und dem geschlossenen Boden definiert ist und wobei eine Querrichtung des Beutels in einer quer zur Längsrichtung verlaufenden Richtung definiert ist, und
- einen Einlass (6, 106), der an der Öffnung definiert ist und sich von der Öffnung und über eine Länge in den Beutel hinein erstreckt, wobei der Einlass (6, 106) ein Antirefluxventil (7, 107) in der Form eines Folienventils ist, bei dem vier Folienlagen in der Längsrichtung zusammengeschweißt sind, wobei eine Breite des Einlasses in der Querrichtung zwischen den Schweißnähten definiert ist und wobei eine Länge eines Einlasses als eine Länge der Folien in der Längsrichtung definiert ist,
**dadurch gekennzeichnet, dass**
eine schmalste Breite des Einlasses (6, 106) zum Einsatz mit einem einen Verbinder (201) umfassenden Urinkatheter (200) geeignet ist und dass das Material des Urinsammelbeutels (1, 101) leicht elastisch ist, so dass die schmalste Breite für eine enge Passung um den Verbinder (201) des Katheters (200) herum sorgt.

2. Urinsammelbeutel nach Anspruch 1, wobei das Material des Urinsammelbeutels (1, 101) bei Bestimmung nach ASTM D882 einen Elastizitätsmodul zwischen 80 MPa und 180 MPa hat.

3. Urinsammelbeutel nach einem der Ansprüche 1 bis 2, wobei die Beziehung zwischen einer schmalsten Breite des Einlasses (6, 106) und der Länge des Einlasses (6, 106) derart ist, dass die Länge weniger als das 3,5-Fache der Breite beträgt.

4. Urinsammelbeutel nach Anspruch 3, wobei die Länge zwischen 4 cm und 8 cm beträgt.

5. Urinsammelbeutel nach einem der Ansprüche 1 bis 2, wobei die Beziehung zwischen einer schmalsten Breite des Einlasses (6, 106) und der Länge des Einlasses (6, 106) derart ist, dass die Länge mehr als das 4-Fache der Breite beträgt.

6. Urinsammelbeutel nach Anspruch 5, wobei die Länge mehr als 10 cm beträgt.

7. Urinsammelbeutel nach einem der vorhergehenden Ansprüche, wobei der Beutel (1, 101) am Einlass (6, 106) mit zwei Klappen versehen ist, um einen Knoten um den Einlass (6, 106) herum zu knüpfen.

8. Urinsammelbeutel nach Anspruch 7, wobei die Klappen vor dem Gebrauch an zerreißbaren Verbindungen am Sammelbeutel (1, 101) angebracht sind.

9. Urinsammelbeutel nach Anspruch 8, wobei die zerreißbaren Verbindungen als Perforationslinien vorgesehen sind.

10. Urinsammelbeutel nach einem der Ansprüche 8 oder 9, wobei die zerreißbaren Verbindungen in am Beutel vorgesehenen Löchern enden, die dazu geeignet sind, dass der Beutel (1, 101) daran aufgehängt oder gehalten wird.

11. Urinsammelbeutel nach einem der vorhergehenden Ansprüche, wobei das Folienventil an einer Seite des Ventils mit Einschnürungen versehen ist.

12. Urinsammelbeutel nach Anspruch 11, wobei die Einschnürungen in einer Position platziert sind, die 0,8 - 0,9 der Länge des Ventils unter der Öffnung des Beutels entspricht.

13. Urinsammelbeutel nach Anspruch 11, wobei Einschnürungen auf einer Höhe von weniger als der halben Länge des Ventils unter der Öffnung des Beutels platziert sind.

14. Urinsammelbeutel nach einem der vorhergehenden Ansprüche, wobei der Beutel mit einer Reißlasche zum Öffnen in einen Auslass (6, 106) aus dem Beutel versehen ist.

15. Urinsammelbeutel nach einem der vorhergehenden Ansprüche, wobei der Beutel (1, 101) mit mindestens einem Haltezipfel zum Halten oder Handhaben des Beutels (1, 101) während des Gebrauchs und des Leerens versehen ist.

16. Urinsammelbeutel nach Anspruch 15, wobei der/die Haltezipfel mit einem Loch zum Aufhängen des Beutels (1, 101) versehen ist/sind.

17. Urinsammelbeutel nach Anspruch 15 oder 16, wobei einer der Haltezipfel gegenüber dem Auslass aus dem Beutel (1, 101) platziert ist.

18. Kit aus Teilen einschließlich eines Urinsammelbeutels nach einem der Ansprüche 1 - 17 und eines Katheters (200) mit einem Auslassteil.

## Revendications

1. Sac collecteur d'urine (1, 101), comprenant:
- une ouverture qui est définie à une extrémité supérieure (2, 102) et un fond fermé qui est défini à une extrémité inférieure opposée (4, 104), dans lequel une direction longitudinale du sac (1, 101) est définie entre l'ouverture et le fond fermé, et dans lequel une direction transversale du sac est définie dans une direction transversale à la direction longitudinale; et
- une entrée (6, 106) qui est définie à l'ouverture et qui s'étend à partir de l'ouverture et sur une longueur dans le sac,
dans lequel l'entrée (6, 106) est une vanne anti-reflux (7, 107) qui se présente sous la forme d'une vanne en feuilles comprenant quatre couches de feuille soudées les unes aux autres dans la direction longitudinale, dans lequel une largeur de l'entrée est définie dans la direction transversale entre les soudures, et dans lequel une longueur d'une entrée est définie comme une longueur des feuilles dans la direction longitudinale,
**caractérisé en ce qu'**une largeur la plus étroite de l'entrée (6, 106) est adaptée pour être utilisée avec un cathéter urinaire (200) comprenant un connecteur (201), et **en ce que** le matériau du sac collecteur d'urine (1, 101) est légèrement élastique de telle sorte que la largeur la plus étroite crée un agencement serré autour du connecteur (201) du cathéter (200).

2. Sac collecteur d'urine selon la revendication 1, dans lequel le matériau du sac collecteur d'urine (1, 101) présente un module d'élasticité qui est compris entre 80 MPa et 180 MPa lorsqu'il est déterminé selon la norme ASTM D882.

3. Sac collecteur d'urine selon l'une quelconque des revendications 1 à 2, dans lequel la relation entre une largeur la plus étroite de l'entrée (6, 106) et la longueur de l'entrée (6, 106) est telle que la longueur soit inférieure à 3,5 fois la largeur.

4. Sac collecteur d'urine selon la revendication 3, dans lequel la longueur est comprise entre 4 cm et 8 cm.

5. Sac collecteur d'urine selon l'une quelconque des revendications 1 à 2, dans lequel la relation entre une largeur la plus étroite de l'entrée (6, 106) et la longueur de l'entrée (6, 106) est telle que la longueur soit supérieure à plus de 4 fois la largeur.

6. Sac collecteur d'urine selon la revendication 5, dans lequel la longueur est supérieure à 10 cm.

7. Sac collecteur d'urine selon l'une quelconque des revendications précédentes, dans lequel le sac (1, 101) est pourvu de deux rabats à l'entrée (6, 106) permettant de nouer un noeud autour de l'entrée (6, 106).

8. Sac collecteur d'urine selon la revendication 7, dans lequel les rabats sont attachés au sac collecteur (1, 101) à des joints déchirables avant leur utilisation.

9. Sac collecteur d'urine selon la revendication 8, dans lequel les joints déchirables sont formés comme des lignes de perforation.

10. Sac collecteur d'urine selon l'une quelconque des revendications 8 ou 9, dans lequel les joints déchirables se terminent dans des trous prévus sur le sac et adaptés pour suspendre ou maintenir le sac (1, 101).

11. Sac collecteur d'urine selon l'une quelconque des revendications précédentes, dans lequel la valve en feuilles est pourvue de resserrements sur un côté de la valve.

12. Sac collecteur d'urine selon la revendication 11, dans lequel des resserrements sont formés à une position qui correspond à 0,8 à 0,9 de la longueur de la valve en dessous de l'ouverture du sac.

13. Sac collecteur d'urine selon la revendication 11, dans lequel des resserrements sont formés à moins de la moitié de la longueur de la valve en dessous de l'ouverture du sac.

14. Sac collecteur d'urine selon l'une quelconque des revendications précédentes, dans lequel le sac est pourvu d'une languette déchirable pour pratiquer une ouverture dans une sortie (6, 106) du sac.

15. Sac collecteur d'urine selon l'une quelconque des revendications précédentes, dans lequel le sac (1, 101) est pourvu d'au moins un lobe de maintien pour maintenir ou manipuler le sac (1, 101) pendant son utilisation et sa vidange.

16. Sac collecteur d'urine selon la revendication 15, dans lequel le(s) lobe(s) de maintien est (sont) pourvu(s) d'un trou pour suspendre le sac (1, 101).

17. Sac collecteur d'urine selon la revendication 15 ou 16, dans lequel un des lobes de maintien est placé en face de la sortie du sac (1, 101).

18. Kit de composants comprenant un sac collecteur d'urine selon l'une quelconque des revendications 1 à 17, et un cathéter (200) présentant une partie de sortie.
